(19) Europäisches Patentamt
European Patent Office
Office européen des brevets

(11) EP 4 093 390 B1

(12) EUROPEAN PATENT SPECIFICATION

(45) Date of publication and mention of the grant of the patent:
**07.05.2025 Bulletin 2025/19**

(21) Application number: **20702095.9**

(22) Date of filing: **23.01.2020**

(51) International Patent Classification (IPC):
***A61K 31/185*** *(2006.01)* ***A61K 31/10*** *(2006.01)*
***A61P 17/02*** *(2006.01)* ***A61P 31/00*** *(2006.01)*
***A61P 31/04*** *(2006.01)* ***A61K 9/00*** *(2006.01)*
***A61K 9/06*** *(2006.01)*

(52) Cooperative Patent Classification (CPC):
(C-Sets available)
**A61K 31/185; A61K 31/10; A61P 17/02; A61P 31/00; A61P 31/04** (Cont.)

(86) International application number:
**PCT/EP2020/051652**

(87) International publication number:
**WO 2021/148124 (29.07.2021 Gazette 2021/30)**

# (54) COMPOSITIONS FOR USE FOR REMOVING NECROTIC OR INFECTED TISSUES FROM BODY SURFACE LESIONS

ZUSAMMENSETZUNGEN ZUR VERWENDUNG ZUR ENTFERNUNG VON NEKROTISCHEN ODER INFIZIERTEN GEWEBEN VON KÖRPEROBERFLÄCHEN

COMPOSITIONS DESTINÉES À ÊTRE UTILISÉES POUR ÉLIMINER DES TISSUS NÉCROTIQUES OU INFECTÉS DE LÉSIONS DE SURFACE CORPORELLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**30.11.2022 Bulletin 2022/48**

(73) Proprietor: **DEBx Medical Holding B.V.**
**1083 HN Amsterdam (NL)**

(72) Inventors:
- **BIGNOZZI, Carlo Alberto**
  **44121 Ferrara (FE) (IT)**
- **COGO, Alberto**
  **36100 Vicenza (IT)**
- **QUINT, Bertus Jozef**
  **1082MA Amsterdam (NL)**

(74) Representative: **Nederlandsch Octrooibureau**
**P.O. Box 29720**
**2502 LS The Hague (NL)**

(56) References cited:
**WO-A1-2014/177530 FR-A1- 2 730 934**
**US-A1- 2016 058 718**

- **COGO ALBERT ET AL: "Restarting the Healing Process of Chronic Wounds Using a Novel Desiccant: A Prospective Case Series", vol. 33, no. 1, 31 December 2020 (2020-12-31), pages 1 - 8, XP009532152, ISSN: 1044-7946, Retrieved from the Internet <URL:https://www.hmpgloballearningnetwork.com/site/wounds/original-research/restarting-healing-process-chronic-wounds-using-novel-desiccant> DOI: 10.25270/WNDS/2021.0108**
- **H. MIKKELSEN ET AL: "Interrelationships between Colonies, Biofilms, and Planktonic Cells of Pseudomonas aeruginosa", JOURNAL OF BACTERIOLOGY, vol. 189, no. 6, 12 January 2007 (2007-01-12), pages 2411 - 2416, XP055739515, ISSN: 0021-9193, DOI: 10.1128/JB.01687-06**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**A61K 31/10, A61K 2300/00;**
**A61K 31/185, A61K 2300/00**

## Description

### Field of the invention

The invention relates to compositions that are usable for removing necrotic or infected tissues from body surface lesions, in particular from skin lesions such as chronic wounds or ulcers.

### Prior Art

It is known that chronic wounds contain microorganisms, in particular bacteria, which replicate and cause a persistent inflammatory response. In particular, the so-called inflammatory cascade produces vasodilation and increases remarkably the haematic flow to the area of the lesion. In a chronic lesion, the continuous presence of virulent microorganisms leads to a massive and persistent inflammatory response, which at the end contributes to damaging the host organism. In fact, a persistent production of inflammation mediators and a constant migration of neutrophil granulocytes occur. The latter release cytolytic enzymes and free radicals in the wound, which are the main culprits for tissue damage. Further, the macrophages are inhibited with the consequent inability of the chronic lesion to self-regenerate. Localized thrombosis may also occur and metabolytes are released with vasoconstricting action that may induce a tissue hypoxia, causing a further bacterial proliferation and tissue destruction. Due to the significant invasiveness of some infecting bacterial species, the microbial component can contribute to aggravating the lesions and increasing the layer of biofilm.

The term "biofilm" defines the thin layer of glycoproteic material that is produced by actively replicating bacteria and that adheres to the bed of the lesion. The biofilm that forms in an infected wound contributes to delaying the healing thereof. In the presence of biofilm, in fact, the conditions are created so that the single microorganisms interact reciprocally by exchanging nutrients and metabolites, so as to constitute proper organized bacterial communities. The biofilms thus act as protected foci of infection and bacterial resistance inside the wound and are able to protect the bacteria from the action of antimicrobial agents such as antibiotics and antiseptics. Normally, the biofilms consist of a plurality of layers of microorganisms and the communication between the microbial cells is a crucial factor for the purposes of the development and maintenance of the biofilm.

The term "chronic cutaneous ulcer" defines the skin lesions that do not progress to healing after at least six weeks of appropriate treatment. Regardless of the aetiology (in fact, diabetic, venous, arteriopathic, vascular and post-traumatic chronic ulcers exist), chronic cutaneous ulcers display some shared pathogenic mechanisms, which make them chronic. Of these pathogenic mechanisms, the most known are: the excessive presence of inflammatory proteins (cytokines and proteases) in the exudate; a persistent colonization of the bottom of the lesion by pathogenic microorganisms; the development of a biofilm that makes the bottom of the lesion inaccessible to drugs and medications. These features of the chronic cutaneous ulcer slow or stop the autolytic process of removal of the non-vital material, the proliferation of neovessels, which is indispensable for the formation of the granulation tissue, and the proliferation of the dermal and epidermal cells.

The chronic cutaneous ulcers, such as for example the chronic cutaneous ulcers of the lower limbs, are a worldwide problem. It is in fact estimated that 1.5% of the world's population is affected by this pathology. As the age is a significant risk factor, the prevalence of the aforesaid chronic cutaneous ulcers is low in children's age and very high in the old age. Also the socioeconomic class, to which the patient belongs, is a risk factor, so that the disease is more widespread in the developing Countries than in the economically more advanced Countries. As also the chronic comorbidities present in the aged population are a risk factor for the development of chronic cutaneous ulcers and as the average age of the population is increasing constantly, the incidence of the chronic cutaneous ulcers is expected to increase progressively in future years.

Methods for treating a chronic wound and methods for treating bacterial infections are known from the prior art and for example described in US2016/0058718A1. The therapeutic treatment of the chronic cutaneous ulcers requires an enormous financial commitment for the health systems. In the USA, about 2 million working days are lost each year because of chronic ulcers. Furthermore, the developed Countries spend about 3 to 5.5% of their total health budget on the treatment of the chronic cutaneous ulcers. The chronic cutaneous ulcers constitute a severe event for the patient, as these pathologies are associated with an increase in morbidity and mortality and are often accompanied by chronic pain that requires the use of major analgesics. Furthermore, the patient becomes dependant on the nursing care that he or she requires and this all entails a significant decline of the patient's quality of life.

It is by now a common conviction amongst the persons skilled in the art that in order to activate the process of healing of a chronic cutaneous ulcer a balance is necessary between the inflammatory process and the regenerative process, so as to take the ulcer to a state that is comparable to that of an acute ulcer. This effect is normally achieved through a known procedure, namely the debridement of the bottom of the ulcer. With this procedure, the non-vital tissues, the stratified secretions on the bottom of the lesion and possibly also the biofilm formed therein are removed surgically.

A drawback of the debridement is that in order for the latter to be effective it has to expose the vital tissue on all the bottom of the ulcer, which creates a significant risk of bleeding for the patient.

Another drawback of the debridement is that this procedure is of surgical type and thus requires the use of a suitably equipped operating theatre and the intervention of specialized staff (doctors and anaesthetists). Consequently, it is necessary to hospitalize the patient. However, the necessary hospitalization and the complexity of performing the debridement increase the costs associated with such a procedure and make this procedure accessible only for a minority of patients.

Non-surgical debridement methods are known, such as the administration of lytic enzyme-based ointments, the negative pressure therapy and some advanced medications. The non-surgical debridement methods, although substantially non-invasive, are unanimously judged as hardly effective or need long periods of application in order to be effective. Therefore there is a strong need to make the treatment of the chronic cutaneous ulcers more effective and less inconvenient and, in particular, to overcome the significant limits imposed by the known (surgical and non-surgical) debridement methods.

From what has been disclosed above, it is clear that the optimum treatment of skin pathologies (chronic cutaneous ulcers) requires a drastic reduction of bacterial contamination, which in turn requires an effective removal of the biofilm and of the necrotic or infected tissues. Nevertheless, the most used surgical method for removing necrotic or infected tissues, i.e. the debridement, has numerous drawbacks (risk of bleeding for the patient, need for hospitalization, complexity and high costs of execution).

Other prior art includes FR2730934, which describes the use of sulphonic acids as bactericidal agents in cosmetic and/or dermatological compositions for topical application. WO2014/177530 describes a home care formulation with antimicrobial activity, the formulation comprising a sulfonic acid derivative and a carboxylic acid, its use as a cleaning agent or disinfection agent or descaling agent.

Therefore, a strong need is felt for new compositions that enable the biofilm and the necrotic or infected tissues to be removed effectively that are present in the chronic cutaneous ulcers.

## Objects of the invention

An object of the invention is to improve the known procedures for removing the biofilm as well as the necrotic or infected tissues present in the skin lesions, in particular in the chronic cutaneous ulcers.

Another object is to provide compositions that are usable for removing the biofilm and the necrotic or infected tissues present in the chronic cutaneous ulcers.

A further object is to provide compositions that can be applied in simple manner to chronic cutaneous ulcers and enable the biofilm and the necrotic or infected tissues to be removed in rapid manner.

## Short description of the invention

According to the invention, a composition as defined in claim 1 is provided, which is usable for removing a biofilm and necrotic or infected tissues from skin lesions. The composition according to the invention comprises a sulfonic acid having the formula $R\text{-}SO_3H$ as active principle, wherein R is an organic alkyl group selected from the group consisting of ethyl and propyl. More precisely, the active principle of the composition according to the invention is selected form the group consisting of ethanesulfonic acid (CAS number: 594-45-6) and 1-propanesulfonic acid (CAS number: 5284-66-2). The composition according to the invention contains at least one of the aforesaid active principles in combination with proton acceptors as defined in claim 1, which are suitable coformulants (chemically inert substances) that are able to adjust, and in particular are able to attenuate, the acidity of the composition.

The composition according to the invention enables the previously mentioned objects to be achieved. In fact, the Inventors have discovered and verified experimentally that a chemical composition containing a strongly hydrophilic substance is able to perform a strongly dehydrating action on the bottom of a chronic cutaneous ulcer. The aforesaid dehydrating action produces a plurality of effects, namely the denaturation of the inflammatory proteins, the drying and the detachment of necrotic biological tissues and above all the elimination of microorganisms, in particular bacteria and fungi.

The composition according to the invention comprises ethanesulfonic or 1-propanesulfonic acid as an active principle, in combination with proton acceptors as defined in claim 1, which are suitable coformulants (chemically inert substances) that are able to adjust, in particular attenuate, the acidity of the composition. The composition according to the invention is able to remove the biofilm and necrotic tissues from infected zones of the skin within a few tens of seconds from the application. The composition can be applied directly on the zone to be treated (chronic cutaneous ulcer) with any means that is suitable for permitting the distribution thereof. After a contact time of a few tens of seconds has elapsed, the composition can be easily removed from the skin or from the mucosa through the use of a simple gauze or by washing the treated skin surface with physiological solution or with a flow of sterile water.

Therefore, by applying the composition according to the invention on a skin lesion it is possible to obtain the same

effects that are obtainable through a surgical debridement, without however the well-known drawbacks connected with the latter procedure. This new and effective therapeutic possibility is usable, with significant benefit for the patient, in cases of slight, medium and high seriousness.

Brief description of the drawings

The invention can be better understood and implemented with reference to the enclosed drawings that illustrate an embodiment thereof by way of non-limiting example in which:

Figure 1 is a photograph of a Petri dish showing a microbial growth inhibition halo around the depositing zone of a sample of a composition (containing ethane sulfonic acid) according to the invention;
Figure 2 is a photograph of a Petri dish showing a microbial growth inhibition halo around the depositing zone of a sample of a composition (containing 1-propanesulfonic acid) according to the invention;
Figures 3A to 3D are photographs showing a wound that was treated and healed after a single application of the composition (containing ethane sulfonic acid) according to the invention;
Figures 4A to 4D are photographs showing a wound that was treated and healed after a single application of the composition (containing 1-propanesulfonic acid) according to the invention;

Detailed description of the invention

The composition according to the invention as defined in claim 1 comprises ethanesulfonic acid or 1-propanesulfonic acid as active principles and is formulated as a gel, which can be easily applied on chronic cutaneous ulcers.
A surprisingly unexpected property of the composition according to the invention is that the latter is able to act in a few tens of seconds on the biofilm and on the necrotic or infected tissues, causing a rapid desiccation thereof and enabling the removal thereof (through washing or sterile gauze) only a few seconds after the application and thus avoiding complicated, painful and costly surgical procedures (debridement).
The action of the composition is due to the release of hydrogen ions ($H^+$) or protons that, possessing a great hydration enthalpy (-1130 KJ/mole), cause the dehydration of the microbial species that make up the biofilm or proliferate in the infected tissues.
This action mechanism is achieved regardless of the microbial species present and makes the composition according to the invention active against any microbial species, whether bacterial, fungal or viral.
The Inventors have carried out researches to identify the most suitable source of protons, namely a source that is such as to enable an effective release of the protons in contact with the skin, with resulting denaturation of the microbial proteins present therein, without however causing damages to the healthy tissue surrounding the lesions. This requires the preparation of appropriate formulations where the release of the protons is optimized, for example by varying the degree of viscosity of the formulations on the basis of the type of lesion for which the use of the composition according to the invention is intended.
The potentially usable sources of protons essentially consist of strong acids (HA), which have to be used in concentrated form, namely in the presence of the least possible amount of water.
This is required because the dissociation of the strong acid into $H^+$ protons and $A^-$anions has to occur in contact with the microbial species, subtracting molecules of water from the latter, which molecules will form the hydration sphere of the H+ protons with release of thermal energy (- 1130 KJ/mole).
The dosage of the H+ protons released by the acid species in concentrated form is problematic as it is not possible to dilute with water, which nevertheless represents the best solvent for solubilizing ethanesulfonic acid or 1-propanesulfonic acid.
As it is known, the acid dissociation constant ($K_a$) relating to the reaction $HA \rightarrow H^+ + A^-$ is defined by the following relation:

$$K_a = [H^+][A^-] / [HA]$$

in which the concentrations in moles/litre of the different species are indicated between square brackets. The negative decimal logarithm of the acid dissociation constant is defined as $pK_a$ ($pK_a = -\log K_a$).
In the following Table 1, the $pK_a$ values are shown (at a temperature of 25°C) of the main strong acids, which dissociate completely, or almost completely, in an aqueous environment.
From Table 1 it can be inferred that the trifluoromethanesulfonic acid is the strongest acid (namely, the acid having the highest $pK_a$ value), whereas the trifluoroacetic acid is the weakest acid:

Table 1

| Acid | Chemical formula | $pK_a$ |
|---|---|---|
| Trifluoromethanesulfonic | $CF_3SO_3H$ | -13 |
| Hydroiodic | HI | -10 |
| Perchloric | HClO4 | -10 |
| Hydrobromic | HBr | -9 |
| Hydrochloric | HCl | -8 |
| Sulphuric | $H_2SO_4$ | -3 |
| Nitric | $HNO_3$ | -1.4 |
| Ethanesulfonic acid | $CH_3CH_2SO_3H$ | -1.3 |
| 1-Propanesulfonic acid | $CH_3CH_2CH_2SO_3H$ | -0.86 |
| Trifluoroacetic | CF3COOH | -0.25 |

Considering the volatility of the hydroiodic, hydrobromic, hydrochloric and trifluoroacetic acids, as well as the oxidizing action of the perchloric and nitric acids, which can cause undesired effects on the skin, the Inventors focused the attention on the sulfonic acid derivatives with ethyl and propyl substituents, which have much lower dissociation constants with respect to sulphuric acid.

The Inventors further provided for the use of ethanesulfonic acid or 1-propanesulfonic acid in combination with suitable proton acceptors (or protonic acceptors) as defined in claim 1, which enable the acidity of the composition according to the invention to be adjusted, and more exactly to be reduced. The protonic concentration has to be reduced because the protons, if they are released in an excessive quantity, may cause the dehydration of the epithelial cells with consequent oedema, erythema, desquamation and tissue necrosis with formation of ulcers. By reducing the concentration of the protons released by the ethanesulfonic acid or 1-propanesulfonic acid, these acids can be effectively used in the treatment of lesions (chronic cutaneous ulcers) in which the biofilm is present for producing a desired dehydrating effect against the contaminating microbial species without however damaging the surrounding healthy tissues.

In one embodiment of the composition, according to the invention, the proton acceptor comprises anhydrous dimethyl sulfoxide (DMSO), which is added to a concentrated solution of ethanesulfonic acid. The DMSO enables the acidity to be reduced of the composition according to the invention by protonation of the oxygen atom thereof.

In another embodiment of the composition according to the invention, the proton acceptor comprises silicon dioxide particles with diameters in the range 200-400 nm. This enables the acidity of the composition according to the invention to be reduced through the protonation of the oxygen present at the surface of the silica particles controlling at the same time the viscosity of the resulting gel.

In a further embodiment of the composition according to the invention the rheology of the gel containing the sulfonic acids can be controlled though addition of tetraethyl orthosilicate, able of binding the silicon dioxide particles.

The aforesaid different embodiments of the composition according to the invention enable an equilibrium to be established between the ethanesulfonic acid or 1-propanesulfonic acid (indicated by HA) and proton acceptors (indicated generally by B). This equilibrium is disclosed by the reaction HA + B ↔ A- + HB and has as a result a reduction of the concentration of the sulfonic acid without introduction of dilution water, maintaining the antimicrobial effect of the composition according to the invention and the efficacy of the latter in removing biofilm and tissues rapidly and painlessly.

The chemical components that are more suitable for preparing the various embodiments of the composition according to the invention are summarized below. Of these, ethanesulfonic acid and 1-propanesulfonic acid are the active principles, whilst all the other listed components are suitable proton acceptors:

Ethanesulfonic acid 97 %; CAS N. 594-45-6

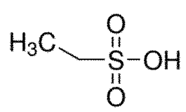

1-Propanesulfonic acid 98%; CAS N. 5284-66-2

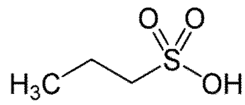

Dimethyl sulfoxide or DMSO or $(CH_3)_2SO$, CAS N. 67-68-5

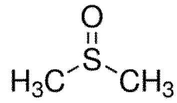

Silicon dioxide or $SiO_2$; CAS 112945-52-5
Tetraethyl orthosilicate or tetraethoxysilane or TEOS or $Si(OC_2H_5)_4$; CAS N. 78-10-4

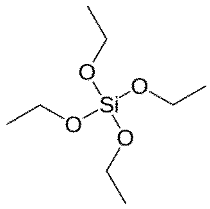

**In** the following Tables 2 and 3, possible qualitative and quantitative formulations of the composition according to the invention are disclosed in greater detail by way of non-limiting examples. The expression "possible qualitative and quantitative compositions" should be understood in the sense that a person skilled in the art can easily and suitably modify each of the formulations of the composition according to the invention (for example, through the addition of pharmacologically acceptable excipients) on the basis of the physical form of administration (solution, cream or gel), provided that the composition always contains ethanesulfonic (first formulation) or 1-propanesulfonic acid (second formulation) as an active principle and at least one suitable proton acceptor.

Table 2

| First formulation (formulation containing ethanesulfonic acid) | |
|---|---|
| Component | Percentage by weight |
| Ethanesulfonic acid | 70-90% |
| Dimethyl sulfoxide | 5-20% |
| $SiO_2$ | 1-8% |
| TEOS | 0.05-2% |

Table 3

| Second formulation (formulation containing 1- propanesulfonic acid) | |
|---|---|
| Component | Percentage by weight |
| 1-Propanesulfonic acid | 70-90% |
| Dimethyl sulfoxide | 5-20% |
| $SiO_2$ | 1-8% |
| TEOS | 0.05-2% |

The procedures for preparing the formulations referred to in the previous Tables 2 and 3 are not disclosed in detail below, because the chemical components of the formulations can be added to the sulfonic acids - namely ethanesulfonic acid and 1-propanesulfonic acid - according to a variable order and nevertheless without altering the properties of the final solution. In fact, the different chemical components are solubilized without reactions intervening that are able to interfere with the phenomenon of the protonation of the proton acceptors.

By way of non-limiting example of the invention, the following Examples are disclosed: a test of *in vitro*

antimicrobial activity of the composition according to the invention (Example 1); a procedure for treating skin lesions by using the composition according to the invention (Example 2); treatment of a wound by using the first formulation (containing ethanesulfonic acid) of the composition according to the invention (Example 3); treatment of a wound by using the second formulation (containing 1-propanesulfonic acid) of the composition according to the invention (Example 4).

Example 1 - Test of *in vitro* antimicrobial activity

The antimicrobial activity of a formulation containing 87% ethanesulfonic acid (first formulation) or a formulation containing 87% 1-propanesulfonic acid (second formulation), both formulations containing 9.8% DMSO, 0.05% Parared, 2.2% SiO2 and 0.95% TEOS, was tested against the following strains of microorganisms (purchased from Diagnostic International Distribution S.p.A.): *Pseudomonas aeruginosa* ATCC 15442, *Staphylococcus aureus* ATCC 6538, *Escherichia coli* ATCC 10536, *Enterococcus hirae* ATCC 10541, *Candida albicans* ATCC 10231. Mixtures of the different strains of microorganisms were prepared, having concentrations expressed as colony forming units (CFU) comprised between $1.5 \times 10^{12}$ - $5.5 \times 10^{12}$ for each species. 100 $\mu$l samples of the mixture were inoculated on Petri dishes containing TSA (Tryptone Soya Agar) solid culture medium. The inoculation was carried out according to a known and standardized analytical method, namely by depositing the liquid sample on the surface of the agar by a micropipette and distributing the liquid sample on the surface of the agar by using sterile glass beads. Subsequently, 50 $\mu$l aliquots of the two formulations of the composition according to the invention were deposited in a central zone of the agar of each Petri dish. The dishes were then incubated at 37°C for 24 h.

Figures 1 and 2 show two Petri dishes, in each of which a 50 $\mu$l aliquot was deposited respectively of the first and second formulation of the composition according to the invention.

After the incubation, the dishes were examined in order to evaluate the microbial proliferation (formation of colonies) and the width of the inhibition halo (namely, the width of the portion of medium in which the microbial proliferation had been inhibited) surrounding the zone of agar on which samples of the two formulations had been deposited. As shown in Figures 1 and 2, in all the Petri dishes clear zones of microbial growth inhibition were observed, surrounding the depositing zones of the preparations according to the invention. The analytical result obtained indicates that the two formulations of the composition according to the invention are able to inhibit the growth of $10^{11}$ CFU of gram-positive bacteria, gram-negative bacteria and of the fungal species *Candida albicans.*

Example 2 - Treatment of patients affected by chronic cutaneous ulcers

The composition according to the invention (first formulation and second formulation) were tested on over 10 patients each, who were volunteers, by applying a treatment protocol comprising the following steps:

- Removing with a sterile gauze the easily removable necrotic materials that are present on the bottom of the ulcer;
- Drying completely the bottom of the ulcer;
- Only in the case of particularly sensitive patients, for whom a painful response is expected, pre-treating the bottom of the ulcer for a time of about 5 minutes with a pack of ointment containing 5% lidocaine;
- Removing the pack, washing the ulcer to remove the lidocaine ointment and drying;
- Applying the composition according to the invention on the bottom of the ulcer and on the surrounding skin for about 1 cm beyond the edge of the ulcer, by using the finger of a hand covered by sterile single-use glove;
- Letting the composition according to the invention to act for about 20 - 30 seconds;
- Washing abundantly with sterile physiological solution;
- Drying with sterile gauze;
- Rubbing the bottom of the ulcer with sterile gauze to remove the desiccated material;
- Leaving on the bottom of the ulcer the possible desiccated material that did not detach itself through the effect of rubbing with the gauze;
- Covering the ulcer with a sterile greasy gauze or with another type of suitable medication (according to the choice of the operator);
- Bandaging the zone according to known procedure;
- Performing subsequent checks at 7-day intervals, or at shorter intervals if deemed necessary.

During each check, the aforesaid protocol provides for proceeding in the following manner:

- Removing bandages and medication;
- Removing progressively, by using normal pliers and scissors and starting from the edges of the lesion, the desiccated material left on the bottom of the ulcer;
- Covering the ulcer with a sterile greasy gauze or with another type of suitable medication (according to the choice of

the operator);
- Bandaging the zone according to known procedure.

When the bottom of the ulcer is covered by granulation tissue, upon complete granulation and according to the choice of the operator, it is possible to graft skin and/or skin substitute in the lesion to complete the healing process.
The treatment protocol disclosed above resulted to be effective in desiccating the bottom of the ulcers in all the treated patients. No complications and/or side effects emerged at the systemic or local level or on the perilesional skin. In all the treated patients the residual desiccated material disappeared progressively at the end of the procedure without the need for an additional intervention and left a granulation tissue on the bottom of the lesion.

Example 3 - Wound treated by a single application of the composition according to the invention, containing ethanesulfonic acid (first formulation)

With reference to Figures 3A-3D, a treatment of a mixed arteriosus-venous ulcer of a lower limb is shown. The treated patient did not respond to previous several types of treatment and the ulcer lasted for almost one year. Figure 3A shows the ulcer before treatment. Figure 3B shows the ulcer immediately after the application of the composition containing ethanesulfonic acid. The desiccant effect of the product is clearly seen, as the slough in the wound bed was instantaneously transformed in a dried layer. The surrounding skin appeared degreased, but without any inflammatory response. No redness, wheals or bullae can be seen. Moreover, a sharp demarcation between the wound bed and the normal skin became apparent. Figures 3C and 3D show respectively the ulcer after 1 and 2 weeks from the initial treatment. It can be noticed that the desiccated material on the wound bed progressively disappeared and the granulation tissue covered the entire wound bed. The disappearance of the desiccated material is due to the rubbing with gauze during follow-up medication. However, an autolytic effect due to reactivation of patient's macrophages, no more inhibited by inflammatory proteins, cannot be ruled out.

Example 4 - Wound treated by a single application of the composition according to the invention, containing 1-propanesulfonic acid (second formulation)

Figures 4A to 4D show the treatment of a post traumatic wound in a patient's leg, with the bottom of the wound bed close to the bone. Figure 4A shows the skin ulcer before treatment: a yellow area can be seen in the upper right part of the wound bed representing the presence of a biofilm. Figure 4B shows the skin ulcer immediately after application of the composition containing 1-propanesulfonic acid and rinsing with saline. The desiccant effect is clearly seen without affecting the surrounding normal skin. The ulcer was then simply covered with a fat gauze. As shown in Figure 4C, full granulation of the wound bed was gradually reached in the subsequent two weeks. Fat gauze was changed weekly. Figure 4D shows that complete healing by spontaneous reepithelization was reached after 5 weeks.
Examples 3 and 4 clearly show that the application of the composition according to the invention (containing ethanesulfonic acid or 1-propanesulfonic acid with proton acceptors) produced in all the cases a complete restoration of the tissues in the lesion, promoting the healing thereof. The treatment protocol disclosed above (see Example 2) can be applied to all the patients, thus avoiding complicated, costly and potentially risky surgical procedures. Furthermore, the treatment with the composition according to the invention can reduce the need to use antibiotic therapies, which are substantially costly and associated with the increasingly rising phenomenon of the antibiotic resistance.
As a person skilled in the art will understand, variations on and/or additions to what has been disclosed above are possible. For example, although the previously disclosed compositions were prepared on laboratory scale, the person skilled in the art is able to provide preparation procedures that are suitable for a production on industrial scale.

## Claims

1. Composition comprising ethanesulfonic acid or 1-propanesulfonic acid, for use in removing biofilm and necrotic or infected tissues from a skin lesion, the composition further comprising a proton acceptor, wherein said proton acceptor is selected from the group consisting of:
dimethyl sulfoxide, silicon dioxide, tetraethoxysilane, and mixtures thereof.

2. Composition for the use according to any one of claims 1, prepared in the form of a gel.

3. Composition for the use according to any one of claims 1 to 2, having the following formulation:

| | |
|---|---|
| Ethanesulfonic acid | 70-90% by weight |
| Dimethyl sulfoxide | 5-20% by weight |
| Silicon dioxide | 1-8% by weight |
| Tetraethoxysilane | 0.05-2% by weight |

**4.** Composition for the use according to any one of claims 1 to 2, having the following formulation:

| | |
|---|---|
| 1-propanesulfonic acid | 70-90% by weight |
| Dimethyl sulfoxide | 5-20% by weight |
| Silicon dioxide | 1-8% by weight |
| Tetraethoxysilane | 0.05-2% by weight |

**5.** Composition for the use according to any one of claims 1 to 4, wherein said use comprises removing said composition from said skin lesion after a contact time of a few tens of seconds has elapsed.

**6.** Composition for the use according to any one of claims 1 to 5, wherein said skin lesion is a chronic cutaneous ulcer.

**7.** Composition for the use according to any one of claims 1 to 6, wherein the proton acceptor reduces the acidity of the composition.

**Patentansprüche**

**1.** Zusammensetzung, die Ethansulfonsäure oder 1-Propansulfonsäure enthält, zur Verwendung bei der Entfernung von Biofilm und nekrotischem oder infiziertem Gewebe von einer Hautläsion, wobei die Zusammensetzung ferner einen Protonenakzeptor umfasst, wobei der besagte Protonenakzeptor aus der Gruppe ausgewählt ist, die aus Folgendem besteht: Dimethylsulfoxid, Siliciumdioxid, Tetraethoxysilan und Mischungen davon.

**2.** Zusammensetzung zur Verwendung nach Anspruch 1, hergestellt in Form eines Gels.

**3.** Zusammensetzung zur Verwendung nach einem der Ansprüche 1 bis 2, die die folgende Formulierung aufweist:

| | |
|---|---|
| Ethansulfonsäure | 70-90 Gew.-% |
| Dimethylsulfoxid | 5-20 Gew.-% |
| Siliciumdioxid | 1-8 Gew.-% |
| Tetraethoxysilan | 0,05-2 Gew.-% |

**4.** Zusammensetzung zur Verwendung nach einem der Ansprüche 1 bis 2, die die folgende Formulierung aufweist:

| | |
|---|---|
| 1-Propansulfonsäure | 70-90 Gew.-% |
| Dimethylsulfoxid | 5-20 Gew.-% |
| Siliciumdioxid | 1-8 Gew.-% |
| Tetraethoxysilan | 0,05-2 Gew.-% |

**5.** Zusammensetzung zur Verwendung nach einem der Ansprüche 1 bis 4, wobei die besagte Verwendung das Entfernen der besagten Zusammensetzung von der besagten Hautläsion nach Ablauf einer Kontaktzeit von einigen zehn Sekunden umfasst.

**6.** Zusammensetzung zur Verwendung nach einem der Ansprüche 1 bis 5, wobei die besagte Hautläsion ein chronisches Hautgeschwür ist.

**7.** Zusammensetzung zur Verwendung nach einem der Ansprüche 1 bis 6, wobei der Protonenakzeptor den Säuregehalt der Zusammensetzung reduziert.

**Revendications**

1. Composition comprenant de l'acide éthanesulfonique ou de l'acide 1-propanesulfonique, destinée à être utilisée pour éliminer le biofilm et des tissus nécrotiques ou infectés d'une lésion cutanée, la composition comprenant en outre un accepteur de protons, où ledit accepteur de protons est choisi dans le groupe constitué par :
le diméthylsulfoxyde, le dioxyde de silicium, le tétraéthoxysilane et des mélanges de ceux-ci.

2. Composition pour l'utilisation selon l'une quelconque des revendications 1, préparée sous la forme d'un gel.

3. Composition pour l'utilisation selon l'une quelconque des revendications 1 à 2, ayant la formulation suivante :

| | |
|---|---|
| Acide éthanesulfonique | 70 à 90 % en poids |
| Diméthylsulfoxyde | 5 à 20 % en poids |
| Dioxyde de silicium | 1 à 8 % en poids |
| Tétraéthoxysilane | 0,05 à 2 % en poids |

4. Composition pour l'utilisation selon l'une quelconque des revendications 1 à 2, ayant la formulation suivante :

| | |
|---|---|
| Acide 1-propanesulfonique | 70 à 90 % en poids |
| Diméthylsulfoxyde | 5 à 20 % en poids |
| Dioxyde de silicium | 1 à 8 % en poids |
| Tétraéthoxysilane | 0,05 à 2 % en poids |

5. Composition pour l'utilisation selon l'une quelconque des revendications 1 à 4, où ladite utilisation comprend l'élimination de ladite composition de ladite lésion cutanée après qu'un temps de contact de quelques dizaines de secondes s'est écoulé.

6. Composition pour l'utilisation selon l'une quelconque des revendications 1 à 5, où ladite lésion cutanée est un ulcère cutané chronique.

7. Composition pour l'utilisation selon l'une quelconque des revendications 1 à 6, où l'accepteur de protons réduit l'acidité de la composition.

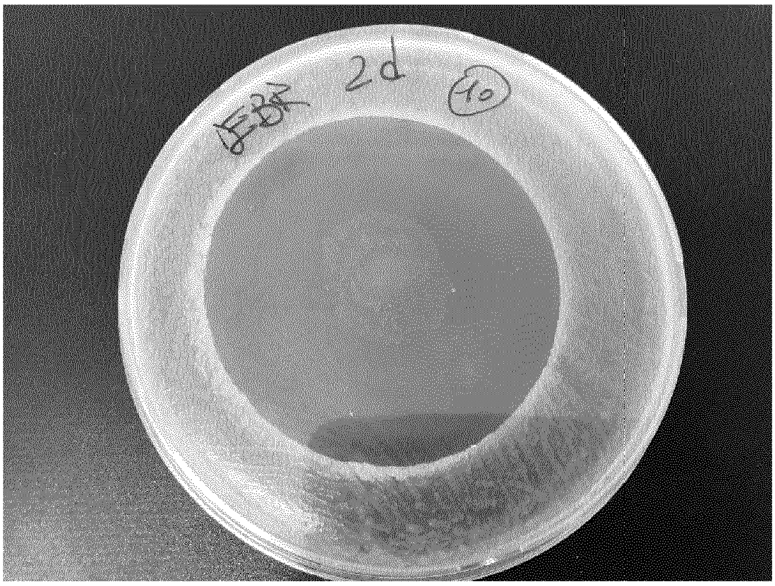

FIG. 1

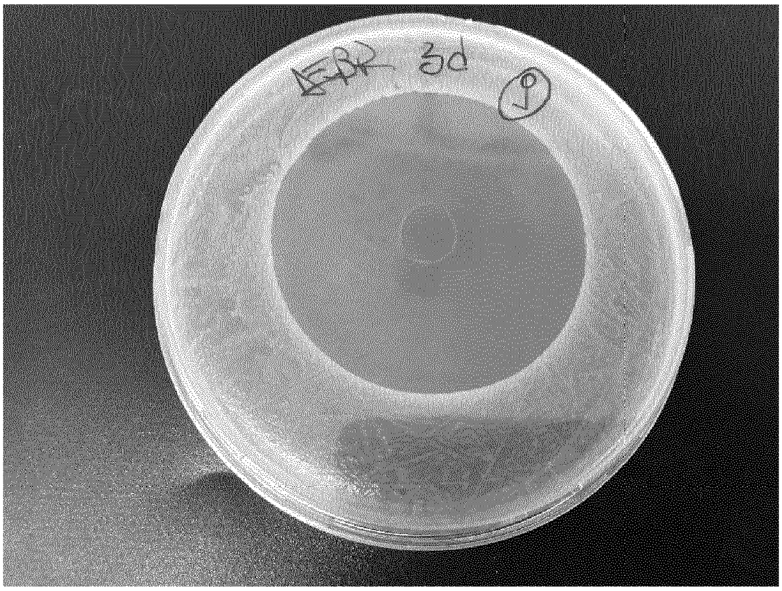

FIG. 2

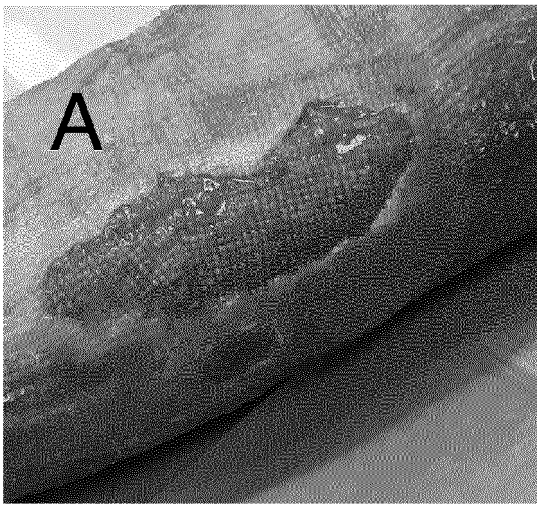


FIG. 3A

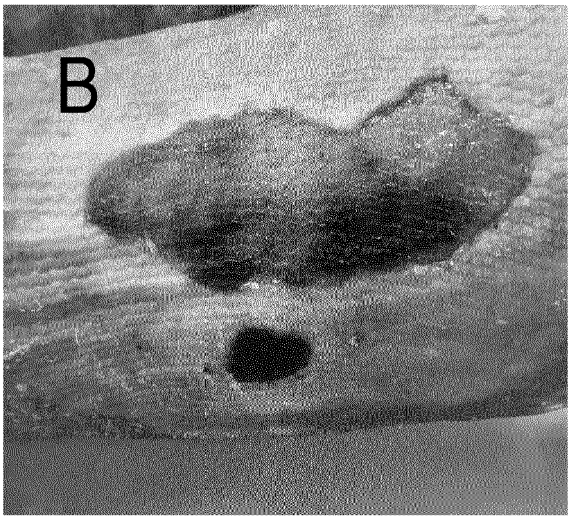


FIG. 3B

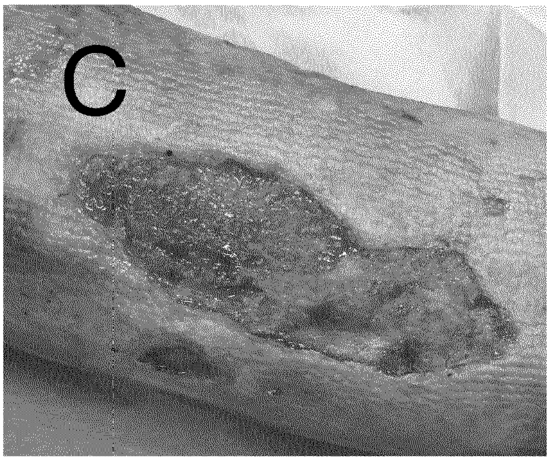


FIG. 3C

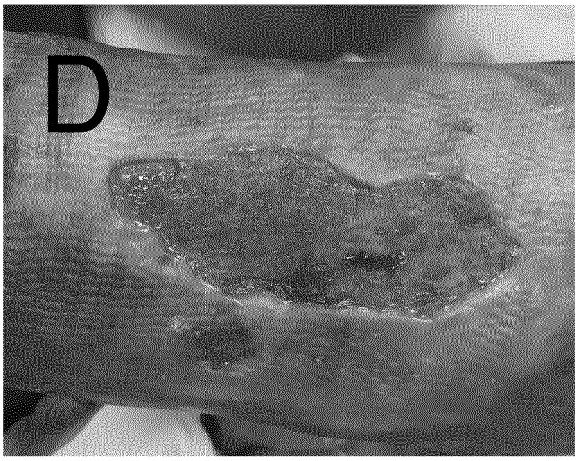


FIG. 3D

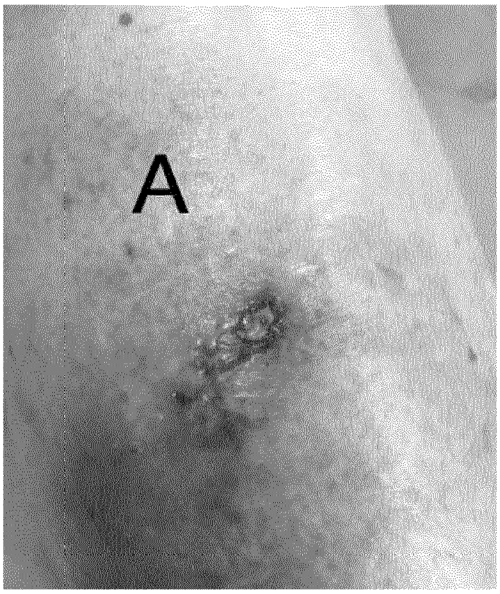


FIG. 4A

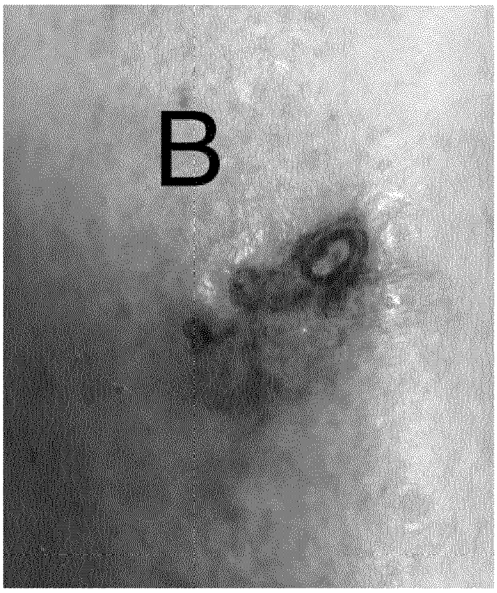


FIG. 4B

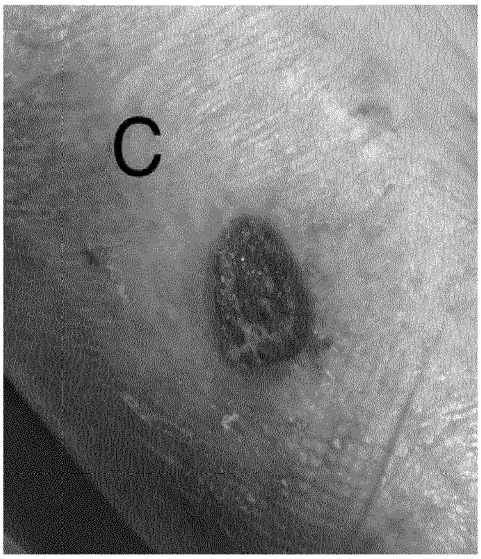


FIG. 4C

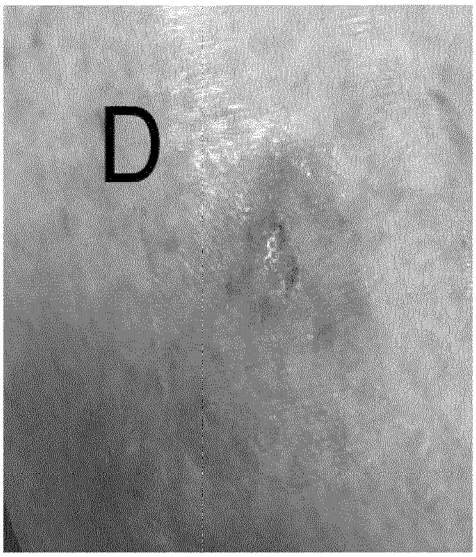


FIG. 4D

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20160058718 A1
- FR 2730934
- WO 2014177530 A


### Non-patent literature cited in the description


- *CHEMICAL ABSTRACTS*, 594-45-6
- *CHEMICAL ABSTRACTS*, 5284-66-2
- *CHEMICAL ABSTRACTS*, 67-68-5
- *CHEMICAL ABSTRACTS*, 112945-52-5
- *CHEMICAL ABSTRACTS*, 78-10-4